# EUROPEAN PATENT SPECIFICATION

(11) **EP 1 780 258 B1**
(45) Date of publication and mention of the grant of the patent: **21.04.2010**
(21) Application number: 06022665.1
(22) Date of filing: 30.10.2006
(51) Int. Cl.: C11B 9/00, C07C 69/92

(54) **Perfume composition**
Riechstoffkomposition
Composition de parfum

(30) Priority: 31.10.2005 JP 2005315855
(43) Date of publication of application: 02.05.2007
(73) Proprietor: Kao Corporation, Chuo-ku Tokyo (JP)
(72) Inventor: Ohno, Satoshi, Sumida-ku Tokyo (JP); Tanaka, Shigeyoshi, Wakayama-shi Wakayama (JP)
(74) Representative: HOFFMANN EITLE

(56) References cited:
- JP-A- 63 190 856
- US-A1- 2005 152 858
- DATABASE WPI Week 199545 Derwent Publications Ltd., London, GB; AN 1995-348502 XP002420356 & JP 07 238296 A (NIPPON OILS & FATS CO LTD) 12 September 1995 (1995-09-12)
- DATABASE WPI Week 199545 Derwent Publications Ltd., London, GB; AN 1995-348503 XP002161632 & JP 07 238297 A (NIPPON OILS & FATS CO LTD) 12 September 1995 (1995-09-12)
- DATABASE WPI Week 200516 Derwent Publications Ltd., London, GB; AN 2005-145404 XP002420357 & JP 2005 015686 A (KAWASAKI K) 20 January 2005 (2005-01-20)

## Description

### FIELD OF THE INVENTION

The present invention relates to perfume compositions containing o-anisic esters and perfume compounds.

### BACKGROUND OF THE INVENTION

Anisic esters are conventionally known as perfume compounds. For example, in Steffen Arctander, "Perfume and Flavor Chemicals", Montclair, N.J., 1969, various p-anisic esters are described as perfume compounds. More specifically, in the above literature, there are described propyl p-anisate (2662) having a gentle delicate sweetly wine-like floral scent, isopropyl p-anisate (2663) having a sweet gentle warmly floral herbal scent, butyl p-anisate (397) having a subtly sweet floral scent, isobutyl p-anisate (398) having a fresh herbal wine fruity scent, tert-butyl p-anisate (399) having a subtly heavy woody floral scent, hexyl p-anisate (1640) having a gentle cherry anise-like fruity floral scent, and heptyl p-anisate (1517) having a subtly sweet fruity wine-like scent.

Also, methyl p-anisate and ethyl p-anisate having a sweet floral scent have been already practically used. Further, there have been proposed perfume compositions for the mouth (JP 2004-18431A) and fruity perfume compositions (JP 2005-15686A) both containing methyl anisate and ethyl anisate as effective ingredients.

On the other hand, o-anisic esters described in the above literature (Steffen Arctander, "Perfume and Flavor Chemicals") are only the methyl o-anisate (1877) having a warmly herbal floral, slightly spicy scent and the ethyl o-anisate (1285) having a sweetly floral, heavy fruity warm scent. Thus, o-anisic esters other than the above two compounds are conventionally unknown as a perfume.

Various perfume substances having a floral scent are known. However, since favorable scents have always changed with the times, it is extremely important in view of the formulation of perfumes to develop and discover new perfume substances. In general, scents of perfume substances are considerably different only by a slight difference in compound structure. In addition, the perfume substances are required to meet various demands such as low cost, good chemical stability and providing a new scent. For example, salicylic esters are known as perfumes having a balsamic scent. However, it is also well known in the art that the salicylic esters tend to be often colored pink by contact with metal ions such as iron ions during production or storage thereof and, therefore, is unsuitable for direct use as a perfume. For this reason, there is a demand to develop chemically stable perfumes having a balsamic scent.

Thus, in order to obtain new perfumes, it is very important to synthesize various compounds which are different in chemical structure from each other even to a slight extent, and carefully examine scents thereof.

JP7238296 (A) relates to a fragrance composition containing a specific 1,1,3,3-tetramethylbutylester. The 1,1,3,3-tetramethylbutylester is said to be useful for imparting lasting odour and flavour to the perfume composition and to have a fruity, floral, woody, grassy, balsamic and/or freesia floral note.

JP63190856 (A) describes the preparation of cis-3-hexenyl p-methoxybenzoate and the use thereof as a perfume raw material. cis-3-Hexenyl p-methoxybenzoate is said to have a leaf-like fragrance and may be blended with various perfume compositions.

### SUMMARY OF THE INVENTION

The present invention relates to the following aspects [1] and [2]:
[1] The use of a composition having an o-anisic ester represented by the general formula (1): 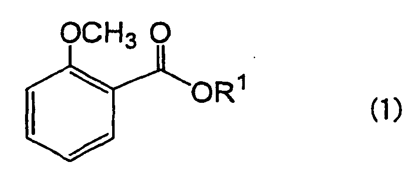 wherein R¹ is a linear or branched alkyl or alkenyl group having 5 to 10 carbon atoms, a cycloalkyl or cycloalkenyl group having 5 to 10 carbon atoms or an arylalkyl group having 7 to 10 carbon atoms, as a perfume.
[2] Cis-3-hexenyl o-anisate represented by the formula (10):

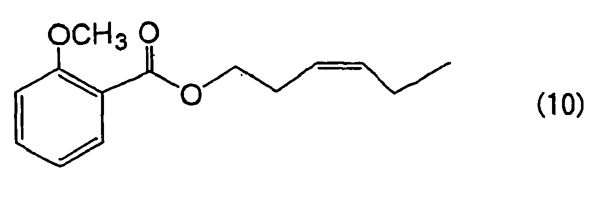

### BRIEF DESCRIPTION OF THE DRAWINGS

Fig. 1 is a chart showing results of ¹H-NMR measurement of cyclohexyl o-anisate obtained in Synthesis Example 1.
Fig. 2 is a chart showing results of FT-IR measurement of cyclohexyl o-anisate obtained in Synthesis Example 1.
Fig. 3 is a chart showing results of ¹H-NMR measurement of cis-3-hexenyl o-anisate obtained in Synthesis Example 2.
Fig. 4 is a chart showing results of FT-IR measurement of cis-3-hexenyl o-anisate obtained in Synthesis Example 2.

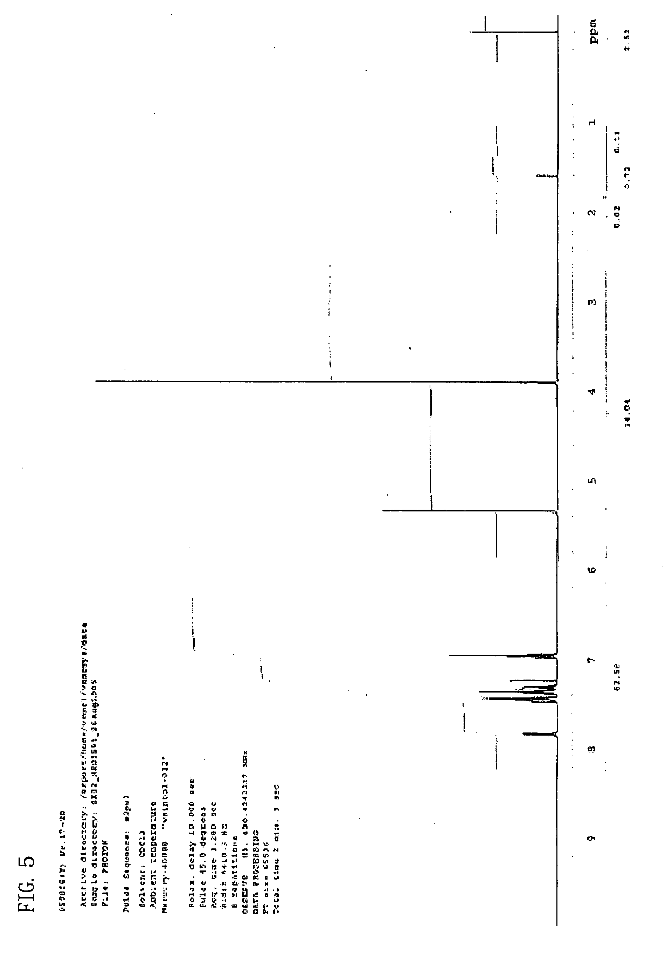

Fig. 5 is a chart showing results of ¹H-NMR measurement of benzyl o-anisate obtained in Synthesis Example 3.
Fig. 6 is a chart showing results of FT-IR measurement of benzyl o-anisate obtained in Synthesis Example 3.

### DETAILED DESCRIPTION OF THE INVENTION

The present invention relates to the use of a composition comprising an o-anisic ester represented by formula (1), as defined above, as a perfume having a floral balsamic scent and to a novel perfume compound.

As a result of the synthesis of various o-anisic esters and the studies on scents and formulations thereof, the inventors have found that perfume compositions containing specific o-anisic esters have an excellent floral balsamic scent, a very excellent tenacity, good chemical stability, and are useful for aromatizing expensive types of products.

That is, the present invention relates to the use of a composition having an o-anisic ester represented by the general formula (1): 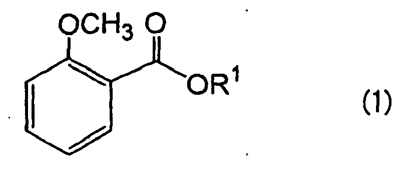 wherein R¹ is a linear or branched alkyl or alkenyl group having 5 to 10 carbon atoms, a cycloalkyl or cycloalkenyl group having 5 to 10 carbon atoms or an arylalkyl group having 7 to 10 carbon atoms as a perfume; and to
cis-3-hexenyl o-anisate represented by the formula (10):

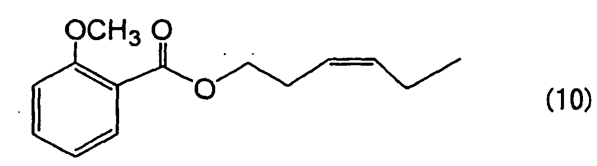

Thus, the perfume composition of the present invention is characterized by containing an o-anisic ester represented by the general formula (1): 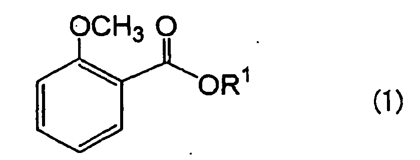 wherein R¹ is a linear or branched alkyl or alkenyl group having 5 to 10 carbon atoms and preferably 6 to 8 carbon atoms, a cycloalkyl or cycloalkenyl group having 5 to 10 carbon atoms and preferably 6 to 8 carbon atoms, or an arylalkyl group having 7 to 10 carbon atoms and preferably 7 to 8 carbon atoms.

Examples of the linear or branched alkyl group having 5 to 10 carbon atoms in the general formula (1) include pentyl, isopentyl, hexyl, 3-methyl-1-pentyl, heptyl, 2- or 3-heptyl, octyl, 2- or 3-octyl, 2-ethylhexyl, nonyl, 2-nonyl, 3,5,5-trimethyl-1-hexyl, 2,6-dimethylheptyl, 3,7-dimethyl-1-octyl and decyl.

Examples of the linear or branched alkenyl group having 5 to 10 carbon atoms in the general formula (1) include prenyl (3-methyl-2-butenyl), 1-penten-3-yl, cis-3-hexenyl, trans-2-hexenyl, trans-3-hexenyl, cis-4-hexenyl, 2,4-hexadienyl, 1-octen-3-yl, cis-6-nonenyl, 2,6-nonadienyl, 1-nonen-3-yl, 9-decenyl, citronellyl (3,7-dimethyl-6-octenyl), geranyl (2-trans-3,7-dimethyl-2,6-octadienyl) and neryl (cis-3,7-dimethyl-2,6-octadienyl).

Examples of the cycloalkyl group having 5 to 10 carbon atoms in the general formula (1) include cyclopentyl, cyclohexyl, cyclohexylmethyl, 2-cyclohexylethyl, 4-isopropylcyclohexyl, 4-tert-butylcyclohexyl, 2-tert-butylcyclohexyl, 4-(1-methylethyl)cyclohexylmethyl, 5-methyl-2-isopropylcyclohexyl, 4-methyl-1-isopropylbicyclo[3.1.0]hexan-3-yl, 1,3,3-trimethylbicyclo[2.2.1]heptan-2-yl, endo-1,7,7-trimethylbicyclo[2.2.1]heptan-2-yl, cycloheptyl, cyclooctyl, cyclodecyl and decahydro-β-naphthyl.

Examples of the cycloalkenyl group having 5 to 10 carbon atoms in the general formula (1) include 2,4-dimethyl-3-cyclohexenylmethyl, 1-methyl-4-isopropenyl-6-cyclohexen-2-yl, 6-methyl-3-isopropenylcyclohexyl, 4-isopropenyl-1-cyclohexenylmethyl and isocyclogeraniol (2,4,6-trimethyl-3-cyclohexenylmethyl).

Examples of the arylalkyl group having 7 to 10 carbon atoms in the general formula (1) include benzyl, 2-phenylethyl and 2-methoxy-2-phenylethyl.

The o-anisic esters represented by the general formula (1) may be produced , for example, by the method represented by the following reaction formula: 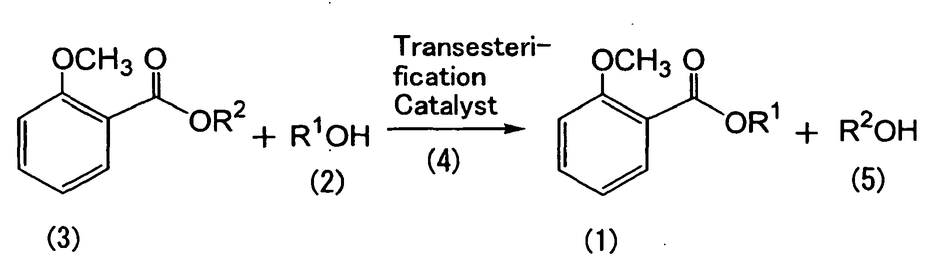 wherein R² is a linear or branched alkyl group having 1 to 4 carbon atoms; and R¹ is the same as defined above.

More specifically, the o-anisic lower alkyl ester (3) such as methyl o-anisate and ethyl o-anisate is transesterified with R¹OH (2) in the presence of the transesterification catalyst (4), thereby obtaining the o-anisic esters represented by the general formula (1). The R²OH (5) produced with progress of the reaction is distilled off out of the reaction system.

The transesterification catalyst (4) used in the above reaction is not particularly limited. In view of a good yield, examples of the suitable transesterification catalyst (4) include sodium methoxide, organic tin catalysts such as di-n-butyl tin diacetate, potassium carbonate and organotitanium catalysts. Among these transesterification catalysts, preferred are sodium methoxide and organic tin catalysts such as di-n-butyl tin diacetate.

Examples of a solvent usable in the above reaction include hydrocarbon-based solvents such as hexane, cyclohexane, benzene, toluene and xylene, and ether-based solvents such as diethyl ether, dibutyl ether and tetrahydrofuran. However, in view of a good productivity, the reaction is preferably conducted in the absence of a solvent.

The amount of the R¹OH (2) used in the above reaction is from 0.7 to 1.7 mol, preferably from 0.8 to 1.5 mol and more preferably from 0.9 to 1.3 mol per 1 mol of the o-anisic lower alkyl ester (3).

The amount of the transesterification catalyst (4) used in the above reaction is from 0.01 to 10-mol%, preferably from 0.05 to 5 mol% and more preferably from 0.1 to 3 mol% on the basis of the o-anisic lower alkyl ester (3).

The temperature used in the above reaction may be appropriately determined according to the kind of solvent used therein, etc., and is usually from 80 to 220°C and preferably from 130 to 180°C. The above reaction may be conducted either under normal pressure or under reduced pressure.

After the reaction reaches a desired equilibrium value, the transesterification catalyst (4) is neutralized with an acid to terminate the reaction, or the reaction mixture is directly subjected to distillation to separate the transesterification catalyst (4) therefrom. Examples of the acid used for the neutralization include inorganic acids such as hydrochloric acid and phosphoric acid, and organic acids such as acetic acid, citric acid and tartaric acid. Among these acids, preferred are inorganic acids.

The reaction solution containing the thus obtained o-anisic esters is distilled under reduced pressure or normal pressure to remove an excessive amount of the R¹OH (2) used therefrom, and then purified by ordinary fractionation or a column chromatography using a column filled with silica gel, etc., to adjust the purity thereof to that usable as a perfume.

The o-anisic esters represented by the general formula (1) may also be produced, for example, by the method represented by the following reaction formula: 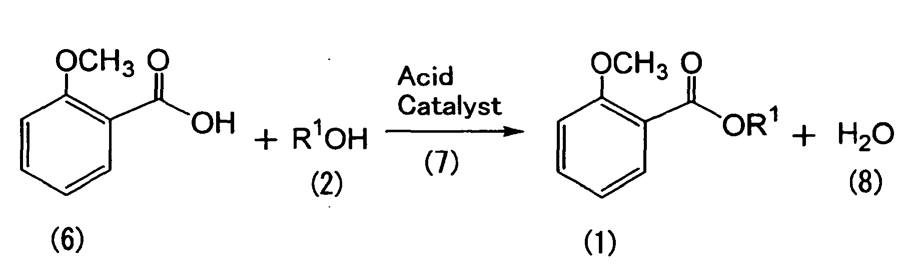 wherein R¹ is the same as defined above.

More specifically, the o-anisic acid (6) and R¹OH (2) are heated in the presence of the acid catalyst (7) to conduct a direct esterification therebetween, thereby obtaining the o-anisic esters represented by the general formula (1).

The acid catalyst (7) used in the above reaction is not particularly limited. In view of a good yield, examples of the suitable acid catalyst include mineral acids such as sulfuric acid and hydrochloric acid, organic acids such as p-toluenesulfonic acid and methanesulfonic acid, and Lewis acids such as boron trifluoride etherate. Among these acid catalysts, especially preferred are sulfuric acid and p-toluenesulfonic acid.

Since the above reaction is generally an equilibrium reaction, it is necessary that water (8) produced is distilled off out of the reaction system. For this purpose, an azeotropic solvent, which may be used, includes hydrocarbon-based solvents such as hexane, cyclohexane, benzene, toluene and xylene, and ether-based solvents such as diethyl ether, dibutyl ether and tetrahydrofuran. However, in view of good productivity, the reaction is preferably conducted in the absence of a solvent.

The amount of the R¹OH (2) used in the above reaction is from 0.7 to 1.7 mol, preferably from 0.8 to 1.5 mol and more preferably from 0.9 to 1.3 mol per 1 mol of the o-anisic acid (6).

The amount of the acid catalyst (7) used in the above reaction is from 0.01 to 30 mol%, preferably from 0.05 to 5 mol% and more preferably from 0.1 to 3 mol% on the basis of the o-anisic acid (6).

The temperature used in the above reaction may be appropriately determined according to the kind of solvent used therein, etc., and is usually from 50 to 220°C and preferably from 80 to 150°C. The above reaction may be conducted either under normal pressure or under reduced pressure.

After the reaction reaches a desired equilibrium value, the acid catalyst (7) is neutralized with an alkali to terminate the reaction. Examples of the alkali used for the neutralization include alkali metal hydroxides such as sodium hydroxide and potassium hydroxide, and alkali earth metal hydroxides such as calcium hydroxide and magnesium hydroxide. Among these alkalis, especially preferred are sodium hydroxide and potassium hydroxide.

The reaction solution containing the thus obtained o-anisic esters is then purified by the same method as described above to adjust a purity thereof to that usable as a perfume.

Meanwhile, among the o-anisic esters represented by the general formula (1), cis-3-hexenyl o-anisate represented by the following formula (10) is a novel compound.

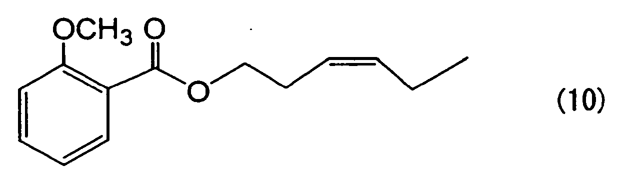

The thus obtained o-anisic esters represented by the general formula (1) and cis-3-hexenyl o-anisate represented by the formula (10) have a novel floral balsamic scent, and may be used alone or in combination with other perfume substances to thereby readily produce perfumes having novel scents.

Examples of the other perfume substances usable in combination with the o-anisic esters include hydrocarbons, alcohols, phenols, esters, carbonates, aldehydes, ketones, acetals, ethers, nitriles, carboxylic acids, lactones, and other natural essential oils or natural extracts.

Examples of the hydrocarbons include limonene, α-pinene, β-pinene, terpinene, cedrene, longifolene and valencene.

Examples of the alcohols include linalool, citronellol, geraniol, nerol, terpineol, dihydromyrcenol, ethyl linalool, farnesol, nerolidol, cis-3-hexenol, cedrol, menthol, borneol, phenylethyl alcohol, benzyl alcohol, dimethylbenzyl carbinol, phenylethyldimethyl carbinol, phenyl hexanol, 2,2,6-trimethylcyclohexyl-3-hexanol and 1-(2-t-butylcyclohexyloxy)-2-butanol.

Examples of the phenols include guaiacol, eugenol, isoeugenol, thymol and vanillin.

Examples of the esters include formic esters, acetic esters, propionic esters, butyric esters, nonenoic esters, benzoic esters, cinnamic esters, salicylic esters, brassilic esters, tiglic esters, jasmonic esters, glycidic esters and anthranilic esters.

Specific examples of the formic esters include linalyl formate, citronellyl formate and geranyl formate. Specific examples of the acetic esters include n-hexyl acetate, cis-3-hexenyl acetate, linalyl acetate, citronellyl acetate, geranyl acetate, neryl acetate, terpinyl acetate, nopyl acetate, bornyl acetate, isobornyl acetate, o-t-butylcyclohexyl acetate, p-t-butylcyclohexyl acetate, tricyclodecenyl acetate, benzyl acetate, stearyl acetate, cinnamyl acetate, diemethylbenzylcarbinyl acetate, phenylethylphenyl acetate and 3-pentyltetrahydropyran-4-yl acetate. Specific examples of the propionic esters include citronellyl propionate, tricyclodecenyl propionate, allylcyclohexyl propionate, ethyl 2-cyclohexyl propionate and benzyl propionate. Specific examples of the butyric esters include citronellyl butyrate, dimethylbenzylcarbinyl n-butyrate and tricyclodecenyl isobutyrate.

Specific examples of the nonenoic esters include methyl 2-nonenoate, ethyl 2-nonenoate and ethyl 3-nonenoate. Specific examples of the benzoic esters include methyl benzoate, benzyl benzoate and 3,6-dimethyl benzoate. Specific examples of the cinnamic esters include methyl cinnamate and benzyl cinnamate. Specific examples of the salicylic esters include methyl salicylate, n-hexyl salicylate, cis-3-hexenyl salicylate, cyclohexyl salicylate and benzyl salicylate.

In addition, specific examples of the brassilic esters include ethylene brassilate; specific examples of the tiglic esters include geranyl tiglate, 1-hexyl tiglate and cis-3-hexenyl tiglate; specific examples of the jasmonic esters include methyl jasmonate and methyl dihyrojasmonate; specific examples of the glycidic esters include methyl 2,4-dihydroxyethylmethylphenyl glycidate and 4-methylphenylethyl glycidate; and specific examples of the anthranilic esters include methyl anthranilate, ethyl anthranilate and dimethyl anthranilate.

Examples of the other esters include commercially available products such as "FRUITATE" (tradename: available from Kao Corp.; ethyltricyclo[5,2,1,0^{2,6}]decane-2-carboxylate).

Examples of the carbonates include commercially available products such as "JASMACYCLAT" (tradename: available from Kao Corp.), "FLORAMAT" (tradename: available from Kao Corp.), and "VIOLIFF" (tradename: available from IFF Inc.).

Examples of the aldehydes include n-octanal, n-decanal, n-dodecanal, 2-methyl undecanal, 10-undecenal, citronellal, citral, hydroxycitronellal, benzaldehyde, phenyl acetaldehyde, phenyl propionaldehyde, cinnamaldehyde, dimethyltetrahydrobenzaldehyde, 4(3)-(4-hydroxy-4-methylpentyl)-3-cyclohexene-1-carboaldehyde ("LYRAL" (tradename) available from IFF Inc.), 2-cyclohexyl propanal, p-t-butyl-α-methylhydrocinnamaldehyde, p-isopropyl-α-methylhydrocinnamaldehyde, p-ethyl-α,α-dimethylhydrocinnamaldehyde, α-amyl cinnamaldehyde, α-hexyl cinnamaldehyde, heliotropin and α-methyl-3,4-methylenedioxyhydrocinnamaldehyde.

Examples of the ketones include α-ionone, β-ionone, γ-ionone, α-methyl ionone, β-methyl ionone, γ-methyl ionone, methyl heptenone, 4-methylene-3,5,6,6-tetramethyl-2-heptenone, amyl cyclopentanone, 3-methyl-2-(cis-2-penten-1-yl)-2-cyclopenten-1-one, methyl cyclopentenolone, rose ketone, carvone, menthone, camphor, acetyl cedrene, isolongifolanone, nootkatone, benzyl acetone, anisyl acetone, methyl β-naphthyl ketone, 2,5-dimethyl-4-hydroxy-3(2H)-furanone, maltol, muscone, civetone, cyclopentadecanone and cyclohexadecanone.

Examples of the acetals include formaldehyde cyclododecylethyl acetal, acetaldehyde ethylphenylpropyl acetal, citral diethyl acetal, phenyl acetaldehyde glycerol acetal and ethyl acetoacetate ethylene glycol acetal.

Examples of the ethers include cedryl methyl ether, anethole, β-naphthyl methyl ether, β-naphthyl ethyl ether, limonene oxide, rose oxide, nerol oxide, 1,8-cineole, rose furan and decahydro-3a,6,6,9a-tetramethylnaphtho[2.1-b]furan.

Examples of the nitriles include geranyl nitrile, citrollenyl nitrile and dodecane nitrile.

Examples of the carboxylic acids include benzoic acid, phenylacetic acid, cinnamic acid, hydrocinnamic acid, butyric acid and 2-hexenoic acid.

Examples of the lactones include γ-decalactone, δ-decalactone, γ-valerolactone, γ-nonalactone, γ-undecalactone, δ-hexalactone, γ-jasmolactone, whisky lactone, coumarin, cyclopentadecanolide, cyclohexadecanolide, ambrettolide, ethylene brassilate, 11-oxahexadecanolide and butylidene phthalide.

Examples of the natural essential oils or natural extracts include orange, lemon, lime, bergamot, vanilla, mandarine, peppermint, spearmint, lavender, camomile, rosemary, eucalyptus, sage, basil, rose, rockrose, geranium, jasmine, ylang ylang, anise, clove, ginger, nutmeg, cardamon, cedar, cypress, vetyver, patchouli, lemongrass and labdanum.

The o-anisic esters represented by the general formula (1) and cis-3-hexenyl o-anisate represented by the formula (10) according to the present invention may be used alone or in combination with the above other perfume substances in optional mixing ratios. The content of the o-anisic esters in the perfume composition is usually 0.001% by mass or higher, preferably 0.01% by mass or higher and more preferably 0.2% by mass or higher in order to impart voluminous sweetness, softness and a high tenacity to a floral scent of the perfume composition, although it is not limited thereto.

### EXAMPLES

The following examples further describe and demonstrate embodiments of the present invention. The examples are given solely for the purpose of illustration and are not to be construed as limitations of the present invention.

### SYNTHESIS EXAMPLE 1: Synthesis of Cyclohexyl o-Anisate (Compound 1 of the present invention)

A four-necked glass flask was charged with 40.23 g (0.242 mol) of methyl o-anisate and 48.27 g (0.482 mol) of cyclohexanol, and while stirring the contents of the flask, 0.430 g (0.00123 mol; 0.51 mol% on the basis of methyl o-anisate) of di-n-butyl tin diacetate was added thereto. The resultant mixture was gradually heated from 151°C to 170°C under normal pressure to react for 6 h while distilling off methanol liberated during the reaction out of the reaction system. After the temperature of the reaction solution reached 170°C, the reaction pressure was reduced to 86.7 kPa, and further the reaction was successively conducted for 6 h while distilling off methanol liberated during the reaction out of the reaction system. The final reaction solution was subjected to gas chromatography to determine a quantity of the thus obtained cyclohexyl o-anisate. As a result, it was confirmed that the reaction yield of the cyclohexyl o-anisate was 99.5%,

Using a 10-stage distillation column, 73.9 g of the final reaction solution was distilled under a reduced pressure of 0.4 to 0.13 kPa to remove unreacted cyclohexanol and methyl o-anisate therefrom, and then successively distilled under a reduced pressure of 0.13 kPa to separate a fraction as the aimed reaction product therefrom, thereby obtaining 42.6 g of cyclohexyl o-anisate represented by the following formula (9). As a result of analyzing the thus obtained fraction by gas chromatography, it was confirmed that the purity of the thus obtained cyclohexyl o-anisate was 99.8%.

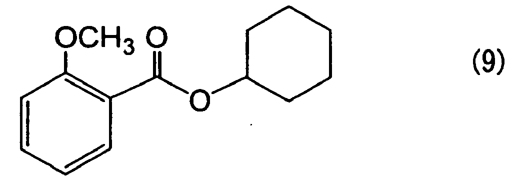

The resultant cyclohexyl o-anisate was subjected to ¹H-NMR analysis using an 400 MHz NMR apparatus available from Varian Inc., and FT-IR analysis using the Fourier transform infrared spectrophotometer "Model: FT-710" available from HORIBA, Ltd., to determine a structure thereof. The results are shown below.
(1) ¹H-NMR (400 MHz, CDCl₃): δ (ppm)
   δ 7.76 (1H, dd J=2.0 Hz, 8.0 Hz), 7.44 (1H, m), 6.96 (2H, m), 5.02 (1H, m), 3.89 (3H, s), 1.94 (2H, m), 1.78 (2H. m), 1.57 (3H. m), 1.41 (3H, m)
(2) FT-IR (neat): (cm⁻¹)
   νₘₐₓ 2937, 2858, 1728, 1601, 1493, 1464, 1300, 1254, 1132, 1080, 1026, 756
(3) Scent: Floral balsamic sweet scent; excellent tenacity

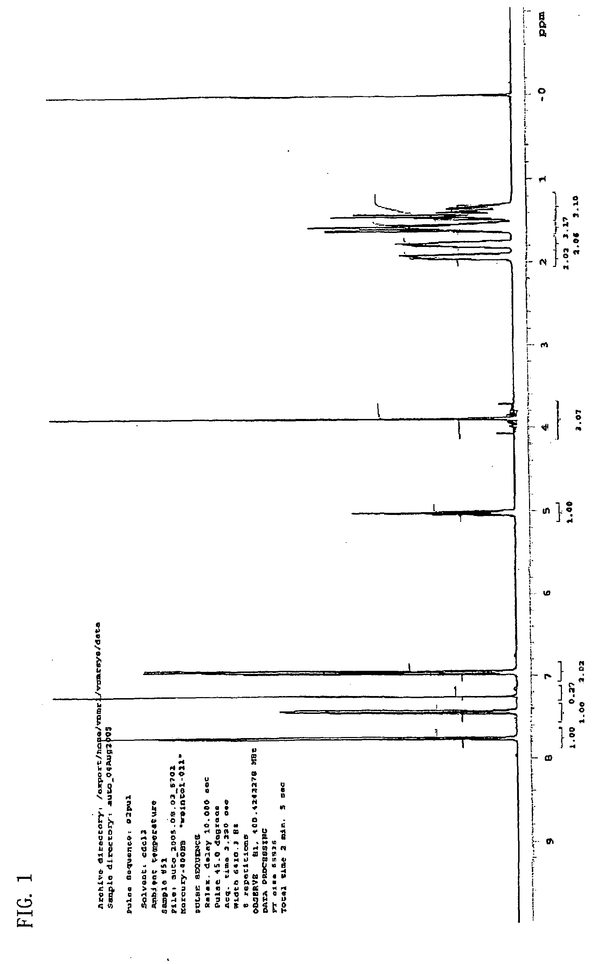

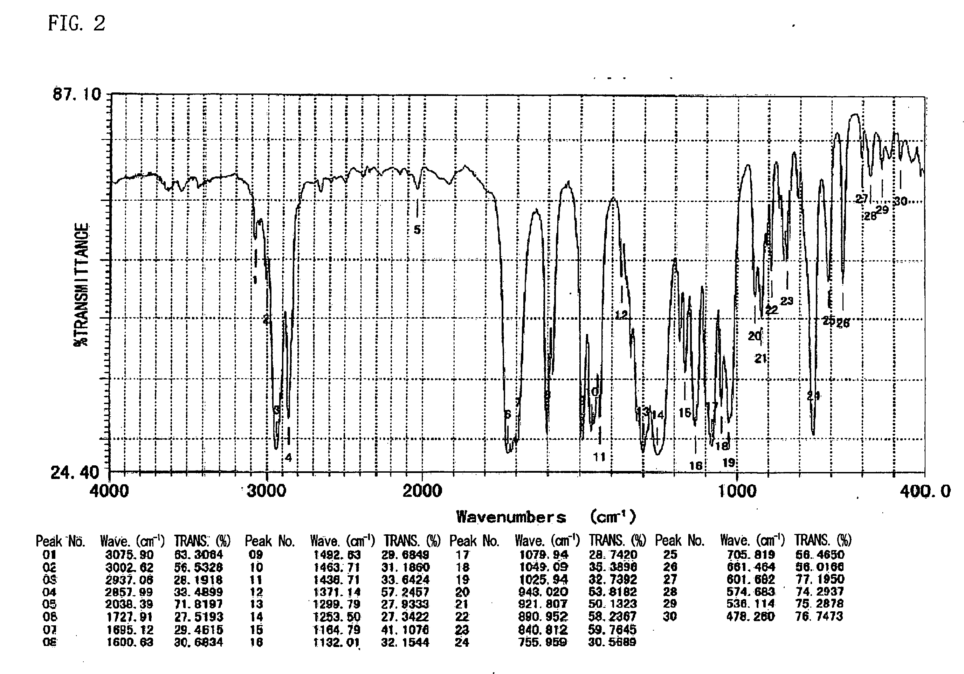

Fig. 1 shows a chart of the ¹H-NMR measurement, and Fig. 2 shows a chart of the FT-IR measurement.

### SYNTHESIS EXAMPLE 2: Synthesis of Cis-3-Hexenyl o-Anisate (Compound 2 of the present invention)

A four-necked glass flask was charged with 58.00 g (0.349 mol) of methyl o-anisate and 70.01 g (0.699 mol) of cis-3-hexanol, and while stirring the contents of the flask, 0.595 g (0.00170 mol; 0.49 mol% on the basis of methyl o-anisate) of di-n-butyl tin diacetate was added thereto. The resultant mixture was gradually heated from 150°C to 170°C under normal pressure to react for 6 h while distilling off methanol liberated during the reaction out of the reaction system. After the temperature of the reaction solution reached 170°C, the reaction pressure was reduced to 86.7 kPa, and further the reaction was successively conducted for 6 h while distilling off methanol liberated during the reaction out of the reaction system. The final reaction solution was subjected to gas chromatography to determine a quantity of the thus obtained cis-3-hexenyl o-anisate. As a result, it was confirmed that a reaction yield of the cis-3-hexenyl o-anisate was 100%.

Using a 10-stage distillation column, 110.01 g of the final reaction solution was distilled under a reduced pressure of 0.4 to 0.13 kPa to remove unreacted cis-3-hexanol and methyl o-anisate therefrom, and then successively distilled under a reduced pressure of 0.13 kPa to separate a fraction as the aimed reaction product therefrom, thereby obtaining 58.64 g of cis-3-hexenyl o-anisate represented by the following formula (10). As a result of analyzing the thus obtained fraction by gas chromatography, it was confirmed that a purity of the thus obtained cis-3-hexenyl o-anisate was 99.7%.

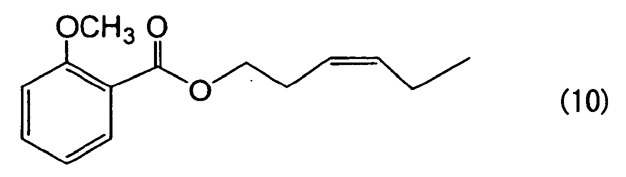

The resultant cis-3-hexenyl o-anisate was subjected to ¹H-NMR analysis and FT-IR analysis in the same manner as described in Synthesis Example 1 to determine a structure thereof. The results are shown below.
(1) ¹H-NMR (400 MHz, CDCl₃): δ (ppm)
   δ 7.77 (1H, dd J=2.0 Hz, 8.0 Hz), 7.45 (1H, m), 6.96 (2H, m), 5.52 (1H, m), 5.40 (1H, m), 4.28 (2H, t J=6.8 Hz), 3.89 (3H, s), 2.50 (2H, m), 2.09 (2H, m), 0.97 (3H, t J=6.8 Hz)
(2) FT-IR (neat): (cm⁻¹)
   νₘₐₓ 2962, 1728, 1601, 1493, 1464, 1302, 1252, 1130, 1082, 756
(3) Scent: Floral balsamic green-like scent; excellent tenacity

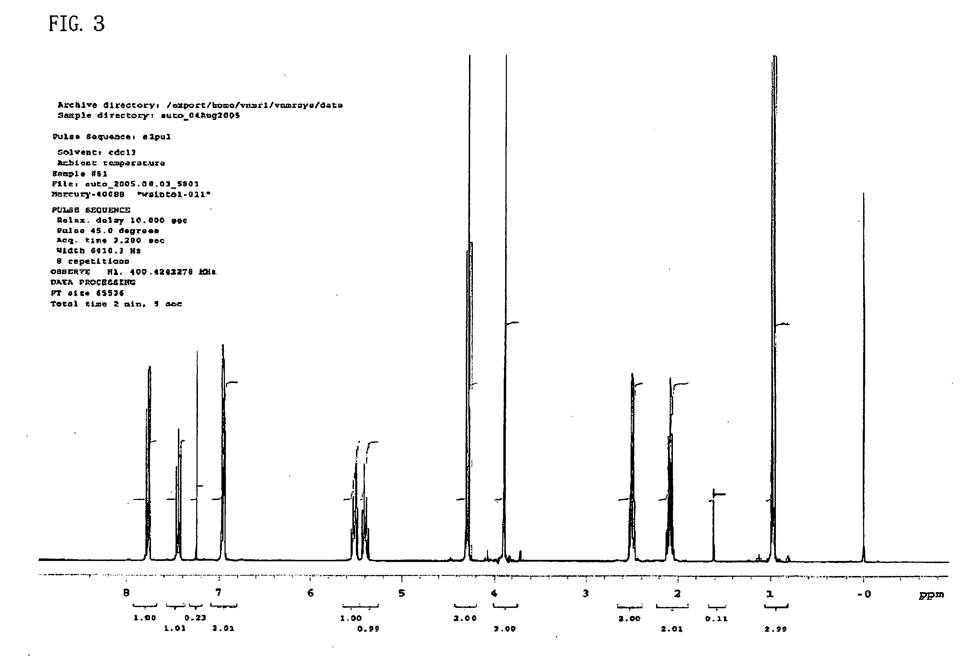

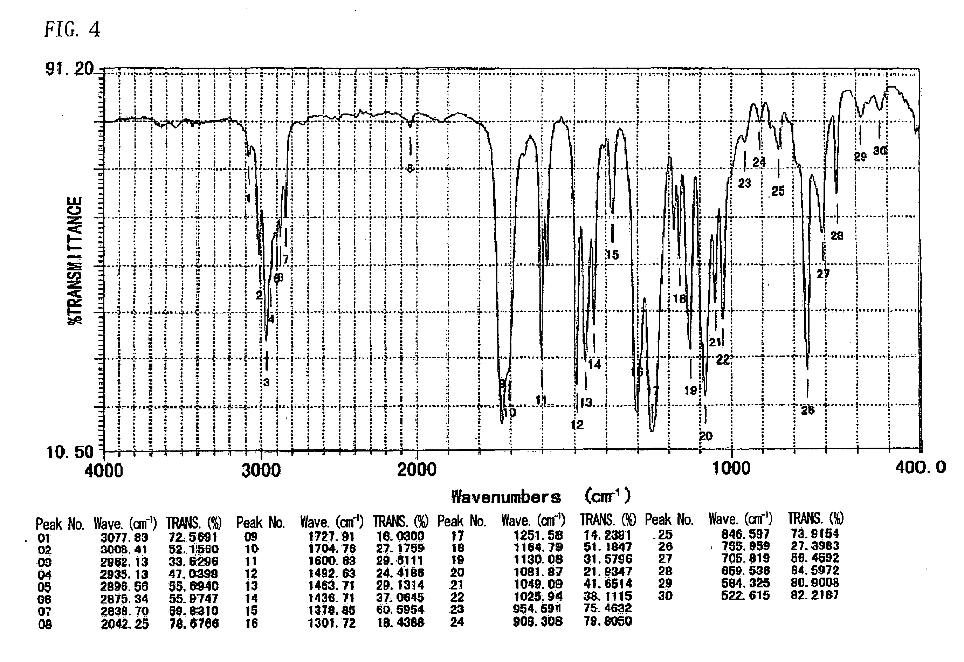

Fig. 3 shows a chart of the ¹H-NMR measurement, and Fig. 4 shows a chart of the FT-IR measurement.

### SYNTHESIS EXAMPLE 3: Synthesis of Benzyl o-Anisate (Compound 3 of the present invention)

A four-necked glass flask was charged with 20.1 g (0.120 mol) of methyl o-anisate and 15.6 g (0.144 mol) of benzyl alcohol, and while stirring the contents of the flask with a stirrer, 0.46 g (0.002 mol) of a 28% sodium methoxide methanol solution was added thereto. The resultant mixture was stirred at 120°C under normal pressure for 2 h. After cooling the mixture, 10 mL of water and 50 mL of diethyl ether were added thereto, and further a 0.1 mol/L hydrochloric acid was added until the pH of a lower layer of the mixture reached 7, thereby separating the mixture into two layers. Water as the lower layer was withdrawn, and an organic layer thus separated was washed with saturated sodium sulfate and then dried with anhydrous magnesium sulfate. After filtering, the resultant reaction solution was subjected to distillation to remove the solvent and unreacted raw materials therefrom, and purified by silica gel-column chromatography, thereby obtaining 5.4 g of benzyl o-anisate represented by the following formula (11). As a result of analyzing the thus obtained sample by gas chromatography, it was confirmed that a purity of the thus obtained benzyl o-anisate was 99.9% (yield: 30%).

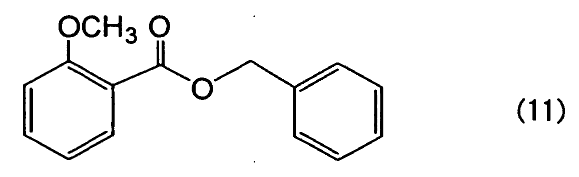

The resultant benzyl o-anisate was subjected to ¹H-NMR analysis and FT-IR analysis in the same manner as described in Synthesis Example 1 to determine the structure thereof. The results are shown below.
(1) ¹H-NMR (400 MHz, CDCl₃): δ (ppm)
   δ 7.82 (1H, dd J=1.6 Hz, 7.6 Hz), 7.45 (3H, m), 7.35 (3H, m), 6.95 (2H, m), 5.34 (2H, s), 3.90 (3H, s)
(2) FT-IR (neat): (cm⁻¹)
   νₘₐₓ 2945, 1732, 1601, 1491, 1464, 1300, 1252, 1130, 1074, 756
(3) Scent: Floral balsamic scent; excellent tenacity

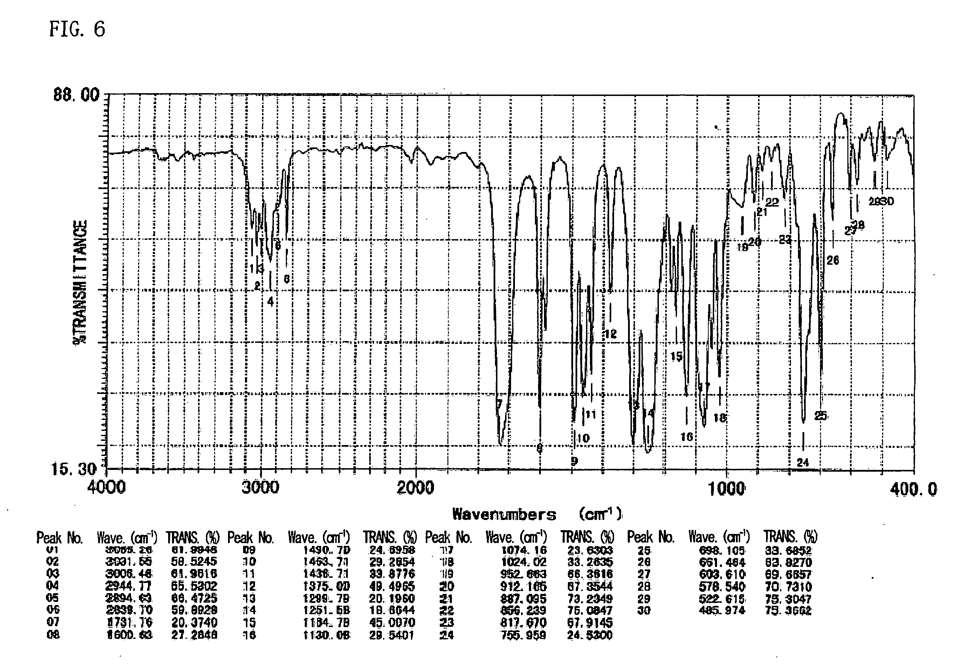

Fig . 5 shows a chart of the ¹H-NMR measurement, and Fig. 6 shows a chart of the FT-IR measurement.

### EXAMPLE 1 AND COMPARATIVE EXAMPLE 1: Floral Sweet Perfume

Using the cyclohexyl o-anisate produced in Synthesis Example 1, there was prepared perfumes having compositions shown in Table 1. In Example 1, 169 parts by weight of dipropylene glycol used in Comparative Example 1 (floral sweet formulated perfume) was reduced to 59 parts by weight, and 110 parts by weight of cyclohexyl o-anisate was used instead, thereby obtaining a floral sweet formulated perfume having an emphasized natural powdery sweet floral scent as well as a high tenacity.

**TABLE 1**

| (unit: weight part) | | |
|---|---|---|
| | Example 1 | Comparative Example 1 |
| Bergamot essential oil | 100 | 100 |
| Linalyl acetate | 50 | 50 |
| Eugenol | 30 | 30 |
| Phenylethyl alcohol | 50 | 50 |
| Phenyl hexanol (product name; available from Kao Corporation) | 50 | 50 |
| LILIAL (product name; available from Givoudan) | 100 | 100 |
| IROTYL (product name; available from Kao Corporation) | 50 | 50 |
| γ-Methyl ionone | 100 | 100 |
| AMBER CORE (product name; available from Kao Corporation) | 100 | 100 |
| Vetiveryl acetate | 30 | 30 |
| SANDALMYSORE CORE (product name; available from Kao Corporation) | 50 | 50 |
| GALAXOLIDE 50IPM (product name; available from IFF Inc.) | 110 | 110 |
| Heliotropin | 10 | 10 |
| AMBROXAN (product name; available from Kao Corporation) | 1 | 1 |
| Cyclohexyl o-anisate (compound 1 of the present invention) | 110 | 0 |
| Dipropylene glycol | 59 | 169 |
| Total | 1000 | 1000 |

### EXAMPLE 2 AND COMPARATIVE EXAMPLE 2: Floral Musky Perfume

Using cyclohexyl o-anisate produced in Synthesis Example 1, there were prepared perfumes having compositions shown in Table 2. In Example 2, 114 parts by weight of dipropylene glycol used in Comparative Example 2 (floral musky formulated perfume) was reduced to 14 parts by weight, and 100 parts by weight of cyclohexyl o-anisate was used instead, thereby obtaining a floral musky formulated perfume having an emphasized musky scent and white flower-imagining scent harmonized therewith as well as a high tenacity.

**TABLE 2**

| (unit: weight part) | | |
|---|---|---|
| | Example 2 | Comparative Example 2 |
| Lemon essential oil | 70 | 70 |
| Cis-3-hexenol | 1 | 1 |
| Helional (product name; available from IFF Inc.) | 15 | 15 |
| FLORAMAT (product name; available from Kao Corporation) | 5 | 5 |
| POIRENATE (product name; available from Kao Corporation) | 5 | 5 |
| Linalyl acetate | 30 | 30 |
| 1-Menthol | 5 | 5 |
| Methyl dihydrojasmonate | 180 | 180 |
| LILIAL (product name; available from Givoudan) | 20 | 20 |
| Linalool | 30 | 30 |
| β-Ionone | 5 | 5 |
| BOISAMBREN FORTE (product name; available from Kao Corporation) | 50 | 50 |
| ISO E SUPER (product name; available from IFF Inc.) | 80 | 80 |
| SANDALMYSORE CORE (product name; available from Kao Corporation) | 100 | 100 |
| Habanolide (product name; available from Firmenich International SA) | 250 | 250 |
| MUSCENONE (product name; available from Firmenich International SA) | 5 | 5 |
| GALAXOLIDE 50IPM (product name; available from IFF Inc.) | 30 | 30 |
| AMBROXAN (product name; available from Kao Corporation) | 5 | 5 |
| Cyclohexyl o-anisate (compound 1 of the present invention) | 100 | 0 |
| Dipropylene glycol | 14 | 114 |
| Total | 1000 | 1000 |

### EXAMPLE 3 AND COMPARATIVE EXAMPLE 3: Floral Fruity Perfume

Using cis-3-hexenyl o-anisate produced in Synthesis Example 2, there were prepared perfumes having compositions shown in Table 3. In Example 3, 261 parts by weight of dipropylene glycol used in Comparative Example 3 (floral fruity formulated perfume) was reduced to 141 parts by weight, and 120 parts by weight of cis-3-hexenyl o-anisate was used instead, thereby obtaining a floral fruity perfume for products having an emphasized fruity sweet scent as well as a high tenacity.

**TABLE 3**

| (unit: weight part) | | |
|---|---|---|
| | Example 3 | Comparative Example 3 |
| Lemon essential oil | 40 | 40 |
| CYCLOVERTAL (product name; available from Kao Corporation) | 2 | 2 |
| FLORAMAT (product name; available from Kao Corporation) | 10 | 10 |
| FRACTON (product name; available from IFF Inc.) | 30 | 30 |
| POIRENATE (product name; available from Kao Corporation) | 20 | 20 |
| γ-Decalactone | 10 | 10 |
| MELUSAT (product name; available from Kao Corporation) | 45 | 45 |
| Ethyl maltol | 1 | 1 |
| Phenyl hexanol | 60 | 60 |
| Rose oxide | 2 | 2 |
| Methyl dihydrojasmonate | 150 | 150 |
| BERUTON (product name; Firmenich International SA) | 10 | 10 |
| Methyl anthranilate | 10 | 10 |
| Linalool | 15 | 15 |
| BOISAMBREN FORTE (product name; available from Kao Corporation) | 50 | 50 |
| SANDALMYSORE CORE (product name; available from Kao Corporation) | 30 | 30 |
| EVERNYL (product name; available from Givoudan) | 2 | 2 |
| Habanolide (product name; available from Firmenich International SA) | 150 | 150 |
| GALAXOLIDE 50IPM (product name; available from IFF Inc.) | 100 | 100 |
| AMBROXAN (product name; available from Kao Corporation) | 2 | 2 |
| Cis-3-hexenyl o-anisate (compound 2 of the present invention) | 120 | 0 |
| Dipropylene glycol | 141 | 261 |
| Total | 1000 | 1000 |

### EXAMPLE 4 AND COMPARATIVE EXAMPLE 4: Floral Spicy Perfume

Using benzyl o-anisate produced in Synthesis Example 3, there were prepared perfumes having compositions shown in Table 4. In Example 4, 475 parts by weight of dipropylene glycol used in Comparative Example 4 (floral spicy perfume) was reduced to 275 parts by weight, and 200 parts by weight of benzyl o-anisate was used instead, thereby obtaining a floral spicy perfume for products having a natural flowery scent with an emphasized spicy sweetness as well as a high tenacity.

**TABLE 4**

| (unit: weight part) | | |
|---|---|---|
| | Example 4 | Comparative Example 4 |
| Bergamot essential oil | 60 | 60 |
| LILIAL (product name; available from Givoudan) | 100 | 100 |
| Jasmine base | 70 | 70 |
| γ-Methyl ionone | 100 | 100 |
| SANDALMYSORE CORE (product name; available from Kao Corporation) | 30 | 30 |
| Habanolide (product name; available from Firmenich International SA) | 120 | 120 |
| MUSCENONE (product name; available from Firmenich International SA) | 10 | 10 |
| Methyl isoeugenol | 35 | 35 |
| Benzyl o-anisate (compound 3 of the present invention) | 200 | 0 |
| Dipropylene glycol | 275 | 475 |
| Total | 1000 | 1000 |

### Chemical Stability (Coloring Property) Test

The compound 1 to 3 obtained in Synthesis Examples 1 to 3, respectively, and salicylic esters known as balsamic perfumes were subjected to a chemical stability test to compare coloring properties thereof in the presence of iron ions with each other.

More specifically, 7.4 mg of a 0.1 wt% ethanol solution of ferric chloride hexahydrate was added to 1 g of each of the compounds of the present invention and the following salicylic esters to observe a coloring property thereof by the visual observation. The results are shown in Table 5.

As a result, it was confirmed that the salicylic esters were instantaneously colored pink when contacted with the iron solution, whereas the compounds of the present invention were free from any color change even upon contacting with the iron solution and chemically stable.

**TABLE 5**

| Compounds of the present invention | Coloring property | Comparative compounds | Coloring property |
|---|---|---|---|
| Cyclohexyl o-anisate | Non-colored | Cyclohexyl salicylate | Colored |
| Cis-3-hexenyl o-anisate | Non-colored | Cis-3-hexenyl salicylate | Colored |
| Benzyl o-anisate | Non-colored | Benzyl salicylate | Colored |

The perfume composition and the perfume compound of the present invention have a floral balsamic scent and exhibit a very high tenacity and a good chemical stability, and can be used alone or in combination with other components in many applications such as household goods, cosmetics and toiletry goods. As a result, the perfume composition and the perfume compound of the present invention can be blended in and applied to aromatic products with various configurations, for example, can be suitably used as an aromatizing component for perfumes, soaps, shampoos and rinses, detergents, cosmetics and aromatic agents.

## Claims

1. Use of a composition comprising an o-anisic ester represented by formula (1), wherein R¹ is a linear or branched alkyl or alkenyl group having 5 to 10 carbon atoms, a cycloalkyl or cycloalkenyl group having 5 to 10 carbon atoms, or an arylalkyl group having 7 to 10 carbon atoms, as a perfume.

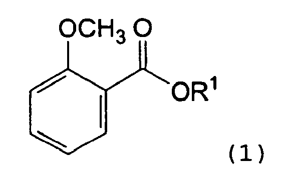

2. The use according to claim 1, wherein R¹ is a linear or branched alkyl or alkenyl group having 6 to 8 carbon atoms, a cycloalkyl or cycloalkenyl group having 6 to 8 carbon atoms or an arylalkyl group having 7 to 8 carbon atoms.

3. The use according to claim 1, wherein R¹ is a cyclohexyl group, a cis-3-hexenyl group or a benzyl group.

4. The use according to claim 1, wherein the o-anisic ester is cis-3-hexenyl o-anisate as represented by the formula (10).

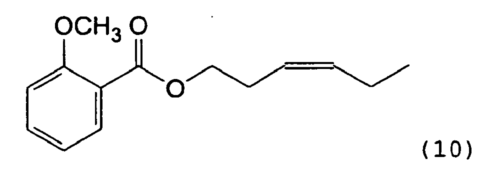

5. Cis-3-hexenyl o-anisate as represented by the formula (10).

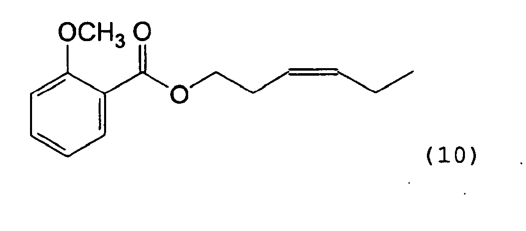

## Patentansprüche

1. Verwendung einer Zusammensetzung, umfassend einen o-Anisester, dargestellt durch die Formel (1), worin R¹ eine lineare oder verzweigte Alkyl- oder Alkenylgruppe mit 5 bis 10 Kohlenstoffatomen, Cycloalkyl- oder Cycloalkenylgruppe mit 5 bis 10 Kohlenstoffatomen oder Arylalkylgruppe mit 7 bis 10 Kohlenstoffatomen ist, als Parfüm:

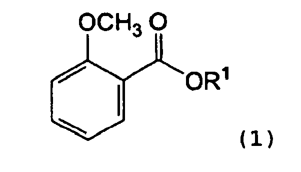

2. Verwendung nach Anspruch 1, worin R¹ eine lineare oder verzweigte Alkyl- oder Alkenylgruppe mit 6 bis 8 Kohlenstoffatom, Cycloalkyl- oder Cycloalkenylgruppe mit 6 bis 8 Kohlenstoffatomen oder Arylalkylgruppe mit 7 bis 8 Kohlenstoffatomen ist.

3. Verwendung nach Anspruch 1, worin R¹ eine Cyclohexylgruppe, eine cis-3-Hexenylgruppe oder eine Benzylgruppe ist.

4. Verwendung nach Anspruch 1, worin der o-Anisester cis-3-Hexenyl-o-anisat ist, dargestellt durch die Formel (10):

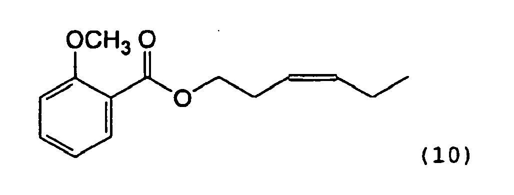

5. cis-3-Hexenyl-o-anisat dargestellt durch die Formel (10) :

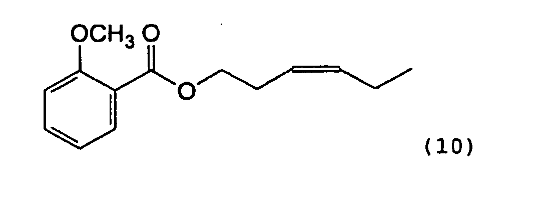

## Revendications

1. Utilisation d'une composition comprenant un ester *o*-anisique représenté par la formule (1), dans laquelle R¹ est un groupe alkyle ou alcényle linéaire ou ramifié possédant 5 à 10 atomes de carbone, un groupe cycloalkyle ou cycloalcényle possédant 5 à 10 atomes de carbone, ou un groupe arylalkyle possédant 7 à 10 atomes de carbone, comme parfum.

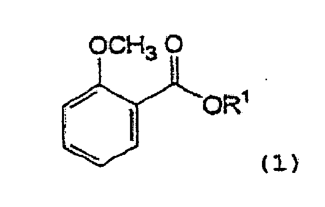

2. Utilisation selon la revendication 1, dans laquelle R¹ est un groupe alkyle ou alcényle linéaire ou ramifié possédant 6 à 8 atomes de carbone, un groupe cycloalkyle ou cycloalcényle possédant 6 à 8 atomes de carbone, ou un groupe arylalkyle possédant 7 à 8 atomes de carbone.

3. Utilisation selon la revendication 1, dans laquelle R¹ est un groupe cyclohexyle, un groupe cis-3-hexényle ou un groupe benzyle.

4. Utilisation selon la revendication 1, dans laquelle l'ester o-anisique est l'*o*-anisate de cis-3-hexényle tel que représenté par la formule (10).

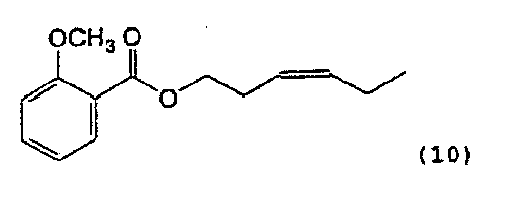

5. *O*-anisate de cis-3-hexényle tel que représenté par la formule (10).
